# EUROPEAN PATENT APPLICATION

(11) **EP 4 374 897 A1**
(43) Date of publication of application: **29.05.2024**
(21) Application number: 22306727.3
(22) Date of filing: 23.11.2022
(51) Int. Cl.: A61M 5/315

(54) **CLOSURE INTEGRITY-PRESERVING KIT, FIXATION CLIP AND PLUNGER ROD FOR A PREFILLED SYRINGE**

(71) Applicant: Becton Dickinson France, 38800 Le Pont-de-Claix (FR)
(72) Inventor: LEHEE, Guillaume, 38340 Voreppe (FR); POUGET, Gaëlle, 38100 GRENOBLE (FR); MILLERET, Anne, 38530 Pontcharra (FR); VAXELAIRE, Jérémie, 38000 Grenoble (FR); GRILLET, Nastasja, 74540 Cusy (FR)
(74) Representative: Germain Maureau

(57) **Abstract**

A prefilled syringe is provided with a fixation clip that, once applied, prevents longitudinal movement of a plunger within a syringe barrel, thereby providing for fixation of the stopper inside the barrel to assure preservation of container closure integrity and maintaining the sterility of the syringe content during conditions such as cold storage and transport, or transport with significant pressure change such as air transport. The fixation clip may include notches thereon accepting a corresponding number of ridges formed on the plunger rod, or alternatively may be formed with an internal cavity to allow sliding over a syringe barrel flange and snapping thereon, and presenting patterns such as protrusions or stops so as to lock the plunger rod, the stopper, and the syringe barrel together. Rotation of the plunger rod up to the desired location liberates the plunger and allows conventional dispensing of medical liquid from the syringe assembly.

## Description

### BACKGROUND OF THE INVENTION

### Field of the Disclosure

The present disclosure relates generally to prefilled syringes. More particularly, the present disclosure relates to a fixation clip and plunger rod for use with a prefilled syringe to preserve the closure integrity of the syringe liquid content during transport or storage.

### Description of the Related Art

Prefilled and prefillable syringes are well known in the medical field for dispensing fluids, such as medications or saline. A conventional syringe typically includes a syringe barrel with an opening at a distal end and a plunger assembly inserted through the opposite proximal end of the barrel. The plunger assembly typically includes an elongated plunger rod extending out of the barrel, and a plunger head or stopper disposed at the distal end of the plunger rod. The stopper or plunger stopper is typically made of elastomeric material, and comprises a body with a tail disposed at its proximal end adapted for attachment to the distal end of the plunger rod, and a head disposed at its distal end. On an external cylindrical wall of the body is defined a plurality of annular outwardly protruding ribs adapted to ensure the container closure integrity of a syringe when the stopper is inserted into the syringe. Moreover, the elongated plunger rod includes a finger flange at its proximal end that, when the plunger assembly is inserted inside the syringe barrel, extends out of the syringe barrel.

After a period of transport and/or storage, during delivery of the medication to a patient, the opening of the syringe barrel on the distal end is adapted for fluid communication with a patient, such as through a needle already attached to the syringe barrel (stacked needle syringe barrel), or through a hypodermic needle fitted at the distal end of the syringe barrel (Luer syringe barrel), or through a Luer-type fitting (needleless access device) fitted at the distal end of the syringe barrel and connected to a fluid line of a patient. Upon the user applying a force to move the plunger assembly through the syringe barrel towards the distal end of the syringe barrel, the content of the syringe is thereby pulled out of the syringe barrel through the opening at the distal end for delivery to the patient. Such an operation is well known in the medical field, and medical practitioners have become well accustomed to the use of such common fluid delivery procedures using prefilled syringes.

Prefilled syringes provide the convenience of rapidly delivering the content to a patient without the need to first aspirate the medication from another container and meter its volume. At the same time, there is a need to maintain sterility and container closure integrity of the medication inside the prefilled syringe, including over an extended period of time, and throughout exposure to various storage and transport conditions. In some circumstances, prefilled syringes are subjected to cold or even freezing temperatures. This may occur deliberately if the content of the prefilled syringe needs to be stored at low temperatures. This may also occur as a result of transport, or storage in cold geographical locations without any temperature control or insulation of the syringe container. Additionally, in some circumstances, prefilled syringes are transported by air and submitted to specific conditions, such as depressurization which may imply low temperatures.

In a typical scenario, once the syringe is filled with a certain predefined volume of the desired drug, the stopper is placed away from the distal end of the barrel by the length that is characterized by the diameter of the barrel, the volume of the liquid, and, at least in some situations, the volume of air between the stopper and the liquid.. Filling and stoppering of the syringe are conventionally done at a predetermined temperature, such as room temperature or at a lower temperature such as 5°C for thermos-sensitive drugs, and under clean environment. Once out of the clean environment, sterilization may be performed. One may define a sterile barrier as being the proximal-most sealing rib of the stopper. The sterile barrier height may also be define as the maximum cumulative distance the stopper can move from its position to the proximal end of the syringe during the storage and/or transport of the syringe before risking to compromise the sterility of the drug and the inside syringe. Significant backward movement of the stopper is highly undesirable as the stopper may move into a non-sterile portion of the barrel which, as a result, can breach the container closure integrity (CCI) and jeopardize the sterility of the syringe drug.

The risk and the extent of unintended stopper movement along the syringe barrel prior to dispensing liquid content from the syringe depends on a number of factors, such as:
- the range of pressure changes (over-pressure and/or depressurization) during the storage and transport of the syringe,
- the range of temperatures during the storage and transport of the syringe,
- inner diameter of the syringe barrel,
- design and material of the stopper,
- gliding performance of the stopper against the specific barrel,
- volume of liquid in the syringe, and
- volume of air (headspace) in the syringe, if any.

The need, therefore, exists for a design of the syringe or its components that can limit or completely prevent the undesirable movement of the stopper inside the barrel during storage and transport of the prefilled syringe and prior to its intended use.

Cold temperature may affect the position of the stopper in the barrel in different ways depending on the ratio of liquid vs air under the stopper (headspace) in the syringe. When a large portion of the syringe is filled with a liquid drug and no air is present between the liquid and the stopper, freezing the liquid causes its expansion by as much as 10% of the volume such as in the case of liquid water turning to ice. In a typical example of a water height of 17 mm (for 1 ml of water stored in a 1 ml syringe), the water height may expand by as much as 1.7 mm, causing a pressure of as high as 900 MPa on the stopper to trigger its movement in a direction away from the tip of the syringe. If the stopper is fixed in place, that much pressure may cause breakage of the barrel and/or may lead to leakage or failure of sterility of the syringe content. The stopper may go back to its initial position after thawing.

On the other hand, large headspace and small liquid volume may cause the stopper to move in the opposite direction during cold storage due to the reduction of air pressure inside the syringe and gas headspace contraction.. The stopper may go back to its initial position after thawing.

Another concern during storage and transport is variation in environment pressure, such as during air transport for instance, or transport/storage in areas with various altitudes (atmospheric pressure depends on altitude). Especially, depressurization may cause the stopper to move in a direction away from the tip of the syringe.

Another concern during storage and transport is an exposure of the syringe packaging to mechanical stress which may accidentally cause application of unintended longitudinal force on the plunger rod, causing the stopper to shift its position within the barrel of the syringe.

The need, therefore, exists for a design of a prefilled syringe capable of preventing the stopper from moving away from the tip of the syringe and towards the flange on the open end of the barrel - at least up until a predefined level of force is applied to the stopper.. The need also exists for a design of the prefilled syringe that prevents the movement of the stopper towards the tip of the syringe. Indeed even if this area of the syringe is sterile, the opposite movement when the system will go back to its initial equilibrium (back to room temperature after cold storage, or back to normal pressure after air transport) will jeopardize the container closure integrity as this area is not guaranteed sterile anymore.

The need also exists for a design of a prefilled syringe capable of providing the above-described limitations on the stopper movement despite variations in filling volume of the liquid content, headspace volume, and other parameters and characteristics during the manufacturing process.

### SUMMARY OF THE INVENTION

The present disclosure provides a syringe plunger rod fixation system configured, once applied, to prevent some or all longitudinal movement of the plunger rod relative to the barrel of the syringe, thereby providing for fixation of the plunger stopper inside the barrel as a way to assure preservation of container closure integrity and maintaining the sterility of the syringe content.

According to one aspect of the invention, a kit with a removable plunger fixation clip is provided to be used with a syringe plunger, wherein the plunger rod features an elongated body with a plurality of spaced apart ridges positioned thereon. The ridges are located on or between the longitudinal walls of the plunger rod, which generally make an X-shape in cross-section and define four quadrants between the walls. Each ridge is made to have a generally triangular shape and spans the space from one quadrant wall to the other. The side of each ridge facing the finger flange at the proximal end of the plunger is positioned at an acute angle to the longitudinal axis of the plunger rod, while the other side is oriented generally perpendicular to the longitudinal axis of the rod. This prevents any movement of the plunger rod towards the tip of the barrel - as explained below in greater detail.

The fixation clip is configured to complement the design of the plunger rod. Its spring-loaded design features a pair of V-shaped arms joined via a hinge in the middle portion of the arms, thereby forming a normally-closed clamp, with each arm having a first end and a second end. The first end of one arm is urged to be next to the first end of the other arm in a normal position. Pushing onto the second end of each arm to move them toward each other causes separation of the first ends. One or both of the first ends features a plurality of notches corresponding in size and spacing to the plurality of ridges on the plunger rod. When the fixation clip is placed on the plunger rod, the notches in the arms of the clip engage with the ridges of the rod so as to lock the position of the clip on the plunger rod. When positioned flush to the barrel flange, the shape of the ridges and corresponding notches of the clip is designed to preclude the stopper from moving toward the distal end of the syringe. Additionally, the shape of the ridges and corresponding notches may preclude the stopper from moving away from the distal end of the syringe, such as during cold storage if the syringe has a large headspace and small liquid volume. In particular, if the stopper is blocked from movement during cold storage, the stopper cannot move distally, resulting in a drop in pressure in the headspace. Subsequently during thawing, the pressure in the headspace increases, but no movement away from the distal end of the syringe occurs as the pressure merely returns to the original equilibrium.

Removal of the fixation clip (by the end-user) is accomplished by moving the second ends of each arm together causing separation of the first ends of each arm, thereby allowing disengagement from the ridges of the plunger rod and sideways removal of the fixation clip from the syringe.

According to another aspect of the invention, a kit with the removable fixation clip is provided to engage with both the plunger rod and the barrel of the syringe, and more specifically with the flange of the proximal end of the barrel. Once the fixation clip is placed on the plunger rod and engaged with the flange of the barrel at the open proximal end thereof, the position of the plunger rod is locked in relation to the barrel. To accomplish this, the fixation clip is further provided with a flange-engaging extension portion having a cutout slot sized to allow the flange of the barrel to be placed inside the cutout slot to interlock with the rest of the fixation clip. The fixation clip also features a curved rest pad configured to be positioned on the outer surface of the cylindrical portion of the barrel so as to better define the position of the fixation clip relative to the plunger rod and the barrel itself. As in the previous aspects of the invention, the fixation clip as described here precludes the stopper from moving toward the distal end of the syringe. It allows the plunger rod to move longitudinally in a direction away from the distal end of the syringe but only if the force exceeds a predetermined threshold. The level of the predetermined force threshold is defined by the value of the acute angle of the ridge side and the number of ridges, as is explained in greater detail below.

According to yet other aspects of the invention, a kit with a further design of the snap-on fixation clip is provided and configured to be removably engaged with both the flange of the barrel as well as the plunger rod finger flange. A slide-in cavity is formed within the disk-shaped fixation clip which is sized to be sufficiently wide to accept the full diameter of the barrel flange therein. Once placed from a side of the flange and positioned at least partially or completely over the flange of the syringe barrel, the snap-on fixation clip is firmly snapped in its place. The fixation clip is also configured to slide into a slot made in the walls of the plunger rod. Once engaged with the clip, the plunger rod cannot move longitudinally along the syringe barrel, thereby locking the position of the stopper inside the barrel. The position of the slot in the walls of the plunger rod is selected to assure a preferred position of the stopper in the barrel once the syringe is filled with the desired volume of the liquid content. To retain the fixation clip in position, a detent or a friction fit between the fixation clip and the barrel flange is used.

According to yet a further aspect of the invention, the retention of the fixation clip in position is assured by twisting the plunger rod after positioning the fixation clip over the barrel flange and through a slot in the plunger rod. A protrusion is provided extending from the fixation clip along the longitudinal axis of the syringe and in a direction towards the thumb-press flange at the proximal end of the plunger rod. A ramp-up is formed on the fixation clip adjacent to the protrusion. Twisting the plunger rod causes the slot of the rod to engage with the ramp-up to snap in place when the slot is off the ramp, thereby fixating all components together. Twisting the rod in the opposite direction frees up the rod from the fixation clip and allows pushing the plunger assembly to inject the medication.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
FIGURE 1 is a perspective view of the kit components according to a first aspect of the present invention,
FIGURE 2 is a close-up perspective view of the same,
FIGURE 3 is a cross-sectional view of the fixation clip over the plunger rod of the first aspect of the invention,
FIGURE 4 is a close-up perspective view of the fixation clip of the first aspect of the invention,
FIGURE 5 is a perspective view of the plunger of the first aspect of the invention,
FIGURE 6 is a perspective view of the kit after assembly according to the second aspect of the present invention,
FIGURE 7 is a dose-up view of the fixation clip attached to the syringe barrel and the plunger rod according to the second aspect of the invention,
FIGURE 8 is another perspective view of the same as in Figure 7.
FIGURE 9 is a perspective view of the fixation clip according to the second aspect of the invention,
FIGURE 10 is a cross-sectional view of the fixation clip taken along a plane A-A in Figure 7,
FIGURE 11 is a perspective view of the fixation clip assembled onto the syringe and the plunger according to a third aspect of the invention,
FIGURE 12 is a side view of the same,
FIGURE 13 is a cross-sectional top view of the same taken along a plane B-B as seen in Figure 12,
FIGURE 14 is a perspective view of the plunger rod of the third aspect of the invention,
FIGURE 15 is a perspective bottom view of the fixation clip of the third aspect of the invention,
FIGURE 16 is a bottom view of the same,
FIGURE 17 is a perspective view of the plunger rod in a different orientation according to the third aspect of the invention,
FIGURE 18 is a perspective view of the fixation clip of the fourth aspect of the invention
FIGURES 19A, 19B, and 19C are top views of the fixation clip at various turning stages of assembly with the plunger rod of the fourth aspect of the invention.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION OF PREFERRED EMBODIMENTS OF THE INVENTION

Other than in the operating examples, or where otherwise indicated, all numbers expressing quantities of ingredients, material properties, and so forth used in the specification and claims and Figures are to be understood as being modified in all instances by the term "about". Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements. Furthermore, when numerical ranges of varying scope are set forth herein, it is contemplated that any combination of these values inclusive of the recited values may be used.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the following discussion, "distal" refers to a direction generally toward an end of a syringe assembly adapted for contact with a patient and/or engagement with a separate device such as a needle assembly or IV connection assembly, and "proximal" refers to the opposite direction of distal, i.e., away from the end of a syringe assembly adapted for engagement with the separate device. For purposes of this disclosure, the above-mentioned references are used in the description of the components of a syringe assembly in accordance with the present disclosure.

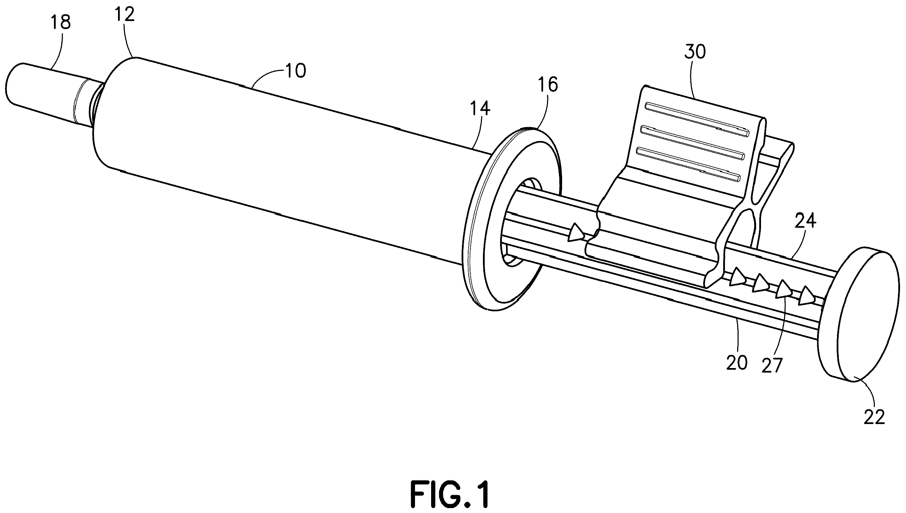

The first aspect of the invention is illustrated in greater detail in Figures 1 through 5. The kit of the present invention generally comprises a syringe barrel 10, a plunger rod 20, and a fixation clip 30. Syringe barrel 10 has a cylindrical elongated body featuring a distal end 12 and a proximal end 14. The distal end 12 includes an outlet opening and/or a mechanism for the attachment of a separate medical device, such as a needleless access device or a needle. The outlet opening at the distal end 12 can be made in a Luer format, or with a needle permanently attached (referred to as a stacked needle)

Syringe barrel 10 may be formed of glass or may be injection molded from a thermoplastic material such as polypropylene, polyethylene, cycloaliphatic polyolefins, cyclo olefin (co)polymers (COC), polyesters, or polycarbonate, for example, according to various techniques, though it is to be appreciated that syringe barrel 10 may be made from other suitable materials and according to other applicable techniques.

Various sizes and volumes of syringe 10 are available ranging from a fraction of a milliliter to tens of milliliters or more. The length and diameter of the syringe barrel 10 may vary according to the volume of the syringe. The proximal end of the syringe barrel 10 is typically open but is intended to be closed off after filling by the drug, accepting a plunger rod 20 with a stopper assembled to the distal end thereof. The barrel of syringe 10 has an outwardly extending flange 16. Although the flange 16 is shown as circular in the drawings, other shapes of the flange 16 are also included in the scope of the present disclosure as the flange 16 is configured for easy grasping by a medical practitioner.

In some designs, the syringe barrel 10 is made with plain sidewalls, while in other designs it may also include markings, such as graduations located on the sidewall, for providing an indication as to the level or amount of fluid contained within the interior of the syringe barrel 10. Such markings may be provided on an external surface of the syringe barrel 10, an internal surface thereof, or integrally formed or otherwise integrated within the sidewall of syringe barrel 10. In other embodiments, alternatively, or in addition thereto, the markings may also provide a description of the contents of the syringe or other identifying information, such as maximum and/or minimum fill lines.

The syringe kit of the invention is useful as a prefilled syringe, and, therefore, may be provided for end use with a fluid, such as a medication or a drug, contained within the interior of syringe barrel 10, prefilled by the pharmaceutical company. In this manner, syringe kit can be manufactured, assembled, prefilled with medication, and sterilized for delivery, storage, and used by the end user, without the need for the end user to fill the syringe with medication from a separate vial prior to use - as described in greater detail below. In such an embodiment, syringe kit may further include a cap 18 or another sealing member positioned at the opening of the distal end 12 to seal the fluid contained in the syringe barrel 10.

A plunger rod 20 is also provided as part of the kit of the invention. The plunger rod 20 has an elongated body extending from a distal end to a proximal end equipped with a finger flange 22. The distal end of the plunger rod 20 is configured for attachment to a stopper (not shown) which may be positioned inside the syringe barrel 10, such as, in one example, by having a threaded attachment 21. The stopper is preferably made of an elastomeric material selected from the group of natural rubber, synthetic rubber, thermoplastic elastomers, or combinations thereof The stopper may be partially or fully coated with, for example, polytetrafluoroethylene (PTFE), ethylene tetrafluoroethylene (ETFE), or the like. The stopper is made to match the internal diameter of the syringe barrel 10 (with a certain compression rate), and is configured for attachment to the plunger rod 20. Although not shown in the drawings, the stopper may be provided as a single body with the plunger rod in other embodiments of the invention. The plunger rod 20 may be manufactured by injection molding from a thermoplastic material, though not necessarily the same material as the syringe barrel 10. For example, the plunger rod 20 may be made of polypropylene, polystyrene, polylactic acid (PLA), or the like.

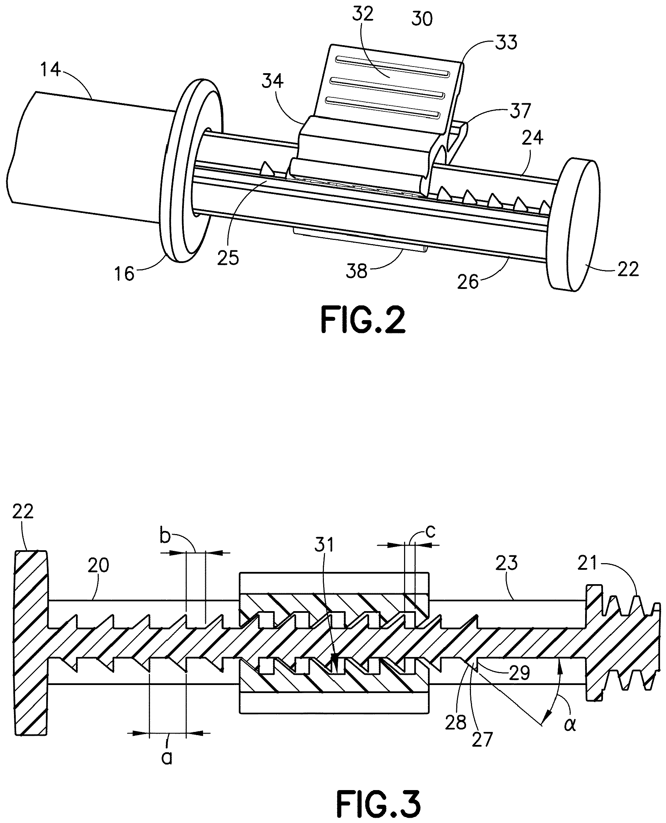

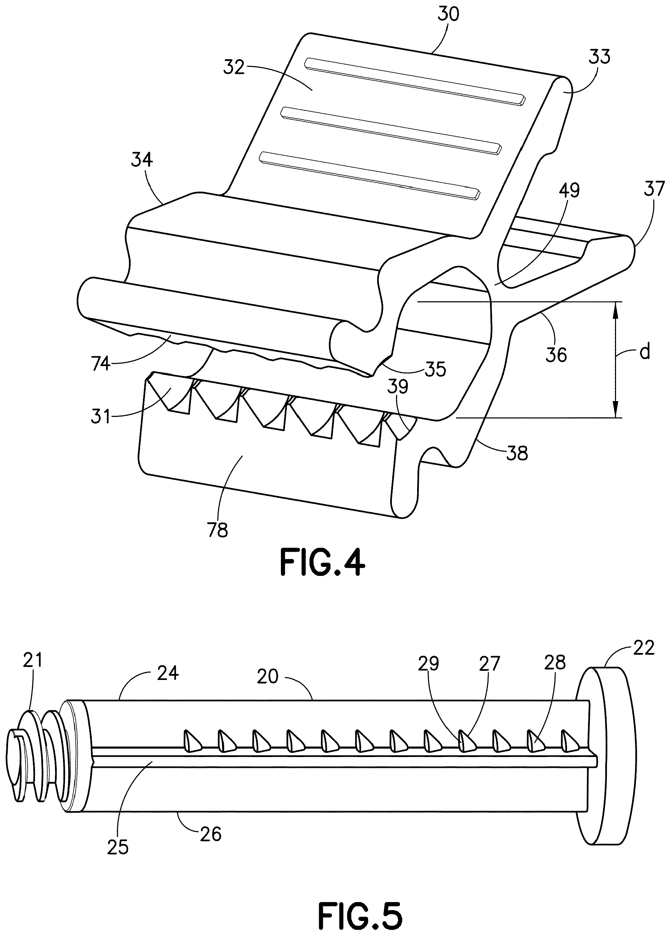

In some designs of the first aspect of the invention, an elongated body of the plunger rod 20 is X-shaped in cross-section and formed by four sidewalls 24, 25, and 26 extending radially away from a center on a longitudinal axis of the plunger rod 20 between the distal end 21 and the proximal end 22 of the plunger rod 20. The side walls are formed at a 90-degree angle to each other defining four quadrants in between. Figure 2 and Figure 5 show three out of four sidewalls 24, 25, and 26 as one example of this design. Other designs of the plunger rod 20 are included in the scope of this disclosure, such as a cylindrical elongated body, an elongated body made with other cross-sectional profiles such as omega-shaped (Ω-shaped), etc.

One or more ridges 27 are formed to extend sideways from the elongated body of the plunger rod 20. The ridges 27 may be formed together with the plunger rod 20 during manufacturing thereof. Anywhere between 1 and 50 or more ridges 27 may be formed on the elongated body of the plunger rod 20, such as at least 1, at least 2, at least 3, at least 4, at least 5, at least 10, at least 15, at least 20, at least 25, at least 30, at least 40, at least 45, at least 50 or more ridges. Ridges 27 may be positioned along a portion of, most of, or the entire length of the plunger rod 20.

Ridges 27 may be formed along one quadrant (as exemplified in Figure 1), two diagonally opposite quadrants (as exemplified in Figure 3), three quadrants, or all four quadrants of the plunger rod 20. When formed along more than one quadrant, ridges 27 are radially aligned with adjacent ridges (see Figure 3, for example) so that turning of the plunger rod does not lead to a longitudinal shift of a position or each ridge 27 from one quadrant to the next along the longitudinal axis of the plunger rod 20. In other embodiments, the ridges 27 may be provided on one or more surfaces or edges of the sidewalls 24, 25, 26.

In one exemplary design as seen in the drawings, a plurality of ridges 27 is spaced apart evenly along the plunger rod 20. One ridge 27 may be placed with a step of a mm (see Figure 3) from another ridge, wherein a may be from about 0.3 mm to about 20 mm, such as every 0.3 mm, every 0.5 mm, every 1 mm, every 1.5 mm, every 2 mm, every 2.5 mm, every 3 mm, every 3.5 mm, every 4 mm, every 4.5 mm, every 5 mm, every 5.5 mm, every 6 mm, every 6.5 mm, every 7 mm, every 8 mm, every 9 mm, every 10 mm, every 11 mm, every 12 mm, every 13 mm, every 14 mm, every 15 mm, every 16 mm, every 17 mm, every 18 mm, every 19 mm, every 20 mm, or any other suitable distance in between as the invention is not limited in this regard.

Each ridge 27 may have the same width and the same space b before the next ridge 27. The space b between the end of one ridge 27 and the beginning of the adjacent ridge may range from 0 mm to about 15 mm, such as 0 mm, 0.5 mm, 1 mm, 1.5 mm, 2 mm, 2.5 mm, 3 mm, 3.5 mm, 4 mm, 4.5 mm, 5 mm, 6 mm, 7 mm, 8 mm, 9 mm, 10 mm, 11 mm, 12 mm, 13 mm, 14 mm, 15 mm or any other suitable length in between.

Each ridge 27 may have a triangular shape as seen in the drawings or any other suitable shape. As best seen in Figure 5, a triangular shape of each ridge 27 may span the space between two adjacent side walls 24 and 25. The body of each ridge 27 is defined by a first side 28 facing the proximal end 22 of the plunger rod 20 and a second side 29 facing the distal end of the plunger rod. While the second side 29 is formed generally perpendicular to the longitudinal axis of the plunger rod 20, the first side 28 is formed at a generally acute angle **α** with the longitudinal axis of the plunger rod, as seen in Figure 3. Angle **α** may be selected from about 10 degrees to about 70 degrees, such as 10 degrees, 15 degrees, 20 degrees, 25 degrees, 30 degrees, 35 degrees, 40 degrees, 45 degrees, 50 degrees, 55 degrees, 60 degrees, 65 degrees, 70 degrees, or any other suitable angle in between.

Fixation clip 30 of the first aspect of the present invention is now described in greater detail with particular reference to Figure 4, as well as referring to Figures 1, 2, and 3. Fixation clip 30 in broad terms comprises a pair of hingedly-connected V-shaped arms 32 and 36 forming a spring-loaded, normally closed clamp seen in Figure 4. In the example in Figure 4, both arms 32 and 36 are attached to each other in the middle portion thereof via a flexible living hinge 39. This design is advantageous as the entire fixation clip 30 may be formed as a single body, for example using injection molding techniques and a suitable thermoplastic material, such as those listed above. Other examples not shown in the drawings may include a design where two separate arms form a fixation clip and are retained together by a metal spring configured to urge the ends of both arms together on one side of the clip. Other examples of the fixation clip not shown in the drawings may include designs to suit customer assembly needs. For example, the arms 32, 36 may not be symmetric to suit particular customer assembly practices.

FIGS. 1 and 2 show the plunger rod 20 partly extracted from the syringe barrel 10, such that the fixation clip 30 is spaced apart from the flange 16 to more clearly show the attachment of the fixation clip 30 to the plunger rod 20. However, in an actual furnished position of the fixation clip 30, a distal end of the fixation clip 30 would bear against the flange 16 of the syringe barrel 10.

Referring again to Figure 4, the thickness of material at location 49 and bulk material properties define the force it takes for separating the arms apart. A first arm 32 has a first end 34 and a second end 33, while a second arm 36 defines a first end 38 and the second end 37. In a normally-closed neutral position of the fixation clip as seen in Figure 4, the distal edge 35 of the first end 34 of the first arm 32 is located in proximity to the distal edge 39 of the first end 38 of the second arm 36. At least one of the distal edge 35, distal edge 39, first end 34, and first end 38 feature at least one or more notches 31 positioned along a length thereof as seen in the drawings. The size, spacing in between, and depth of notches 31 are made to correspond to that of respective plunger rod ridges 27, as can be appreciated best from Figure 3. In that case, positioning of the fixation clip 30 over the plunger rod 20 causes at least one of the ridges 27 to be located within at least one of the corresponding notches 31.

In some designs of the first aspect of the invention, the size of each notch 31 is an exact match to the size of each ridge 27, but made in reverse, wherein the height and width of the ridge 27 define the depth and width of the notch 31. In other designs, the internal space of each notch 31 may be somewhat wider than the width of the ridge 27, as seen in Figure 3, defining a slack or a gap of the length c in between each ridge 27 when positioned in the notch 31. Gap length c may vary from 0 to 5 mm, such as 0mm, 0.5 mm, 1 mm, 1.5 mm, 2 mm, 2.5 mm, 3 mm, 3.5 mm, 4 mm, 4.5 mm, 5 mm, or any other suitable distance. One advantage of providing some space inside notch 31 is to allow the plunger rod 20 a small degree of movement up to the limits of the length c while the fixation clip 30 is positioned over the plunger rod 20. This is useful to compensate for some of the component assembly variability during manufacturing, such as accumulation of tolerances from molding and assembly processes.

While the second end 33, 37 of each arm 32 and 36 may be made having a generally straight shape, the first end 34, 38 of each arm 32, 36 is made to have an arch shape - as seen in Figure 4. This is done to allow the fixation clip 30 to go over the opposite sidewalls 24 and 26 of the plunger rod 20 and permit the distal edges 35 and 39 to come close together. The extent of the arch depth d (see Figure 4), in this case, is dependent on the size of the corresponding opposite sidewalls 24 and 26 of the plunger rod 20. Depending on the size of the plunger rod 20, dimension d may be from about at least 3 mm to about 30 mm or more, such as at least 3 mm, at least 4 mm, at least 5 mm, at least 6 mm, at least 7 mm, at least 9 mm, at least 10 mm, at least 12 mm, at least 14 mm, at least 16 mm, at least 18 mm, at least 20 mm, at least 22 mm, at least 24 mm, at least 26 mm, at least 28 mm, or at least 30 mm, as defined by the corresponding plunger rod of the kit of the present invention.

Each of the first ends 34, 38 of each arm 32, 36 may have a sloped leading edge 74, 78. The sloped leading edges 74, 78 engage the plunger rod 20 as the fixation clip 30 is brought toward the plunger rod 20 in a direction transverse to the longitudinal axis of the plunger rod 20. The force of contact between the plunger rod 20 and the sloped leading edges 74, 78 causes the first ends 34, 38 of the arms 32, 36 to separate, allowing the fixation clip 30 to fully seat on the plunger rod 20. Thus, an operator and/or an assembly equipment do not need to squeeze the second ends 33, 37 of the arms 32, 36 to install the fixation clip 30 on the plunger rod 20.

During the manufacturing process of the prefilled syringe, a syringe barrel 10 may be first filled with a liquid medication or drug and then assembled with a plunger stopper and the plunger rod 20 by insertion into the internal space of the syringe barrel 10. Empty syringe assembly is referred here to as a prefillable syringe, while after the syringe barrel is filled with liquid content and assembled with the plunger assembly, it is referred to as a ready-to-use prefilled syringe.

A number of medical liquids may be loaded into the prefillable syringe assembly of the invention, including various types of saline, such as isotonic saline and hypertonic saline, any suitable liquid medication, vaccine, or any other liquid medicament or medical liquid content that is intended to treat, prevent, or alleviate the symptoms or side effects of a disease or a condition, as defined by medical professionals.

Sterilization of the prefillable syringe and/or prefilled syringe containing a suitable medical liquid content is accomplished as part of the manufacturing process using any number of sterilization techniques. For example, the prefillable syringe may be provided sterile on one side, and the stopper may be provided sterile on the other side. Filling and assembly of the prefilled syringe may be performed in a sterile environment, which may eliminate the need for sterilization after assembly. Nevertheless, in some embodiments, a sterilization process, such as steam sterilization, may be performed after filling and assembly of the prefilled syringe.

After filling the syringe barrel and assembling the plunger assembly, the plunger rod 20 protrudes from the open proximal end 14 of the syringe barrel 10, fixation clip 30 is placed onto the plunger rod 20 such that it extends sideways beyond the boundaries of the syringe barrel 10. The position of the fixation clip is selected to have one side thereof to be next to the flange 16 of the syringe barrel 10. This position assures that the plunger rod 20 is precluded from moving inward, thereby preventing leakage.. The distal end 12 of the syringe barrel 10 is capped and sealed with a cap 18 to further isolate the internal volume of the syringe barrel 10 from the outside environment and protect the sterility of the liquid content.

Prior to the intended use of the prefilled syringe and dispensing of the liquid medication to the patient, a medical operator is instructed to first remove the fixation clip 30 from the plunger rod 20 by pushing the second ends 33, 37 of both arms 32, 36 of the clip 30 together, thereby causing the first ends 34, 38 thereof to separate apart. As the distal edges 39 and 35 are moved away from each other, ridges 27 are released from the corresponding notches 31 and the fixation clip 30 may be moved sideways away from the plunger rod 20. Once the fixation rod 30 is removed, medical liquid may be dispensed by pushing on the flange 22 to move the stopper toward the distal end 12 of the syringe barrel 10.

The fixation clip 30 may be discarded if the entire volume or a desired portion of the medical liquid content is dispensed at that time. If liquid dispensing is done in stages, the fixation clip 30 may be optionally reapplied to the plunger rod 20 and located next to the flange 16, thereby better preserving the position of the stopper in the syringe barrel 10.

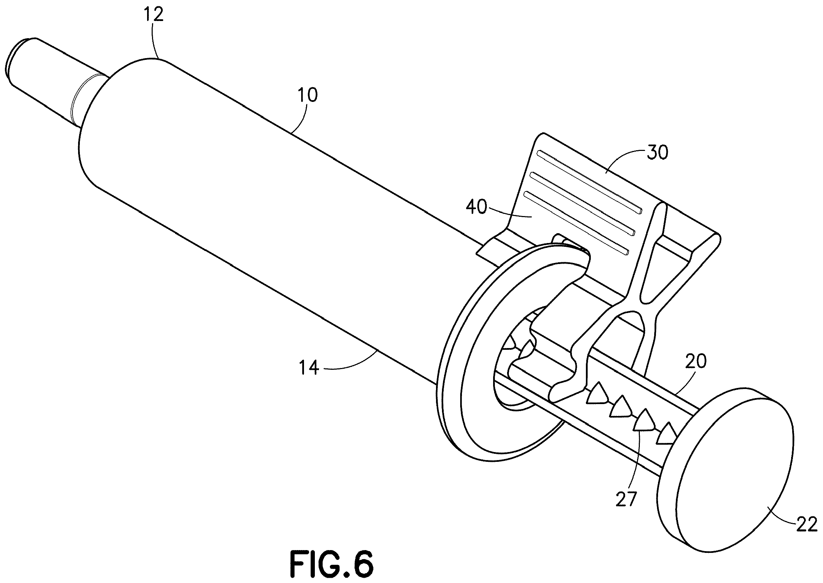

Details of the second aspect of the present invention are now described and best seen in Figures 6 through 9. While the first aspect of the invention (shown in FIGS. 1-5) is capable of preventing the plunger rod 20 and the stopper from moving toward the distal end 12 of the syringe barrel 10, during transport and storage of the prefilled syringe for instance, it may be not able to prevent the unintended movement thereof in the opposite direction, which is away from the distal end 12. A more definitive lock of the plunger rod 20 with the syringe barrel 10 is needed to exclude the risk of any significant movement of the stopper in any direction while inside the syringe barrel 10 so as to better preserve the sterility of the medical liquid content thereof.

The fixation clip 30 of the second aspect of the present invention features a flange-engaging extension portion 40 extending from at least one or both second ends 33 and 37 of the arms 32 and 36 on the same side. Extension 43 is made together with and extends from the second end 33, while extension 47 is made together with and extends from the second end 37 in the same general direction. A cutout slot 44 is provided between the extension portion 40 and the main body of the fixation clip 30. The depth and width of the cutout slot 44 are selected to be at least sufficient to accept the flange 16 inside thereof upon positioning of the fixation clip 30 of the second aspect of the invention over the syringe barrel 10.

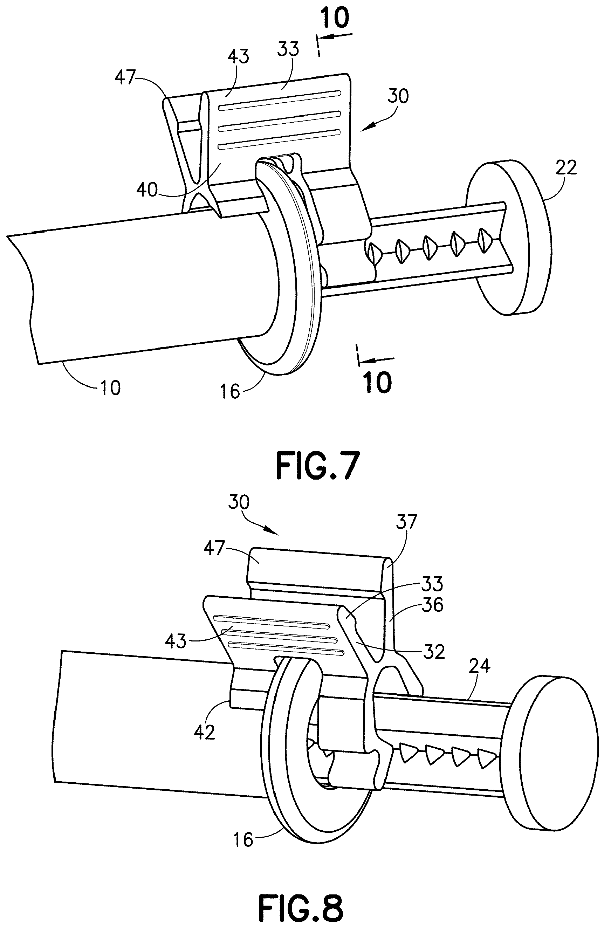

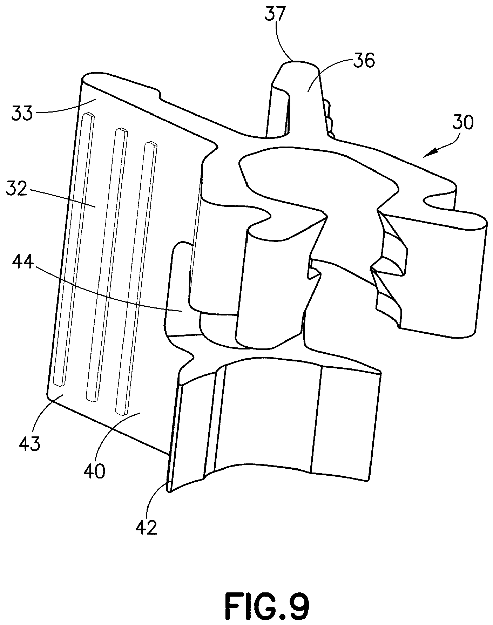

A curved rest pad 42 may be optionally provided as part of the extension 40 as seen best in Figure 9. Dimensions and extent of curvature of the rest pad 42 are selected to match the outside diameter of the syringe barrel 10 such that the rest pad 42 is placed onto the external surface of the syringe barrel 10 when the fixation clip 30 is placed over the plunger rod 20 as seen in Figures 7 and 8.

The use of the fixation clip 30 of the second aspect of the present invention is similar to that of the first aspect, in that it is placed to engage with the plunger rod 20 in the same manner. The difference is that upon placement, the fixation clip 30 of the second aspect is also engaging with the flange 16 of the syringe barrel 10, thereby locking the position of the plunger rod 20 in respect to the syringe barrel 10 and not allowing the stopper to move in either one of the directions toward or away from the distal end 12 of the syringe barrel 10.

While the shape of the ridge is selected to completely prevent the movement of the plunger rod 30 toward the distal end 12 of the syringe barrel 10 (with the exception of small slack as defined by dimension c), the fixation clip 30 of the second aspect of the invention is further useful in allowing the plunger rod 20 to move away from the distal end 12 of the syringe barrel 10, but only when the outward force on the stopper exceeds a predetermined threshold level.

As with the embodiment shown in FIG. 5, the first side 28 of each ridge 27 is formed at a generally acute angle **α** with the longitudinal axis of the plunger rod, as seen in Figure 3. Angle **α** may be selected from about 10 degrees to about 70 degrees, such as 10 degrees, 15 degrees, 20 degrees, 25 degrees, 30 degrees, 35 degrees, 40 degrees, 45 degrees, 50 degrees, 55 degrees, 60 degrees, 65 degrees, 70 degrees, or any other suitable angle in between. The angle **α** plays an important role in defining the force threshold at which the plunger rod 20 is able to move away from the distal end of the syringe barrel 10.

When pressure changes are encountered (such as due to cold storage and/or altitude changes), expansion of the liquid content of the syringe may apply significant pressure on the stopper of the plunger rod 20. In response, the plunger rod 20 may cause the ridges 27 to apply some sideways force on the notches 31 depending on the angle **α** and other dimensions of the ridge/notch geometrical arrangement, as well as mechanical resiliency characteristics of the material used to make the fixation clip 30. All of the above-mentioned factors together define a threshold force, which if exceeded would allow the arms of the fixation clip 30 to open up and the plunger rod 20 to move away from the distal end 12 of the syringe barrel 10. That is, exceeding the threshold force causes the fixation clip 30 to temporarily break engagement with the ridges 27, shift distally along the plunger rod 20 by a distance equal to the length c between ridges 27, and reengage the ridges 27 at a more distal location on plunger rod 20. The width of ridge 27 would also define the extent of outward movement of the plunger rod 20 so that the fixation clip 30 would be allowed to close back over the plunger rod 20 to have the notches 31 engage with an adjacent set of ridges 27. In various designs of the fixation clip and the corresponding plunger rod 20 of the kit of the present invention, the level of the threshold force required for the plunger rod 20 to move away from the distal end of the syringe barrel 10 is selected to exceed at least 0.5 kgf or more so as to keep the plunger rod 20 in place when the force on the stopper is below the threshold level. In further designs, the level of the threshold is selected to be at least 1 kgf, at least 1.5 kgf, at least 2 kgf, at least 2.5 kgf, at least 3 kgf, or more as the invention is not limited in this regard. Higher threshold force assures better stability of the stopper inside the syringe barrel 10 as can be appreciated by those skilled in the art.

The relative position of fixation clip 30 to plunger rod 20 may also serve as an indicator that the content of the syringe barrel 10 is compromised and should not be given to a patient. For example, significant movement of the fixation clip from its initial position to its final position onto the plunger rod 20 before removal of the fixation clip 30 is indicative of unintended pressure buildup in the syringe barrel 10. Such pressure buildup may be indicative that the content of the syringe barrel 10 is compromised and should be discarded. For example, an indicating mark could be provided on the plunger rod 20 at a predetermined distance (e.g. 2 mm) from an initial, safe position of the fixation clip 30. If the fixation clip 30 moves beyond the indicating mark, the prefilled syringe is assumed to be compromised.

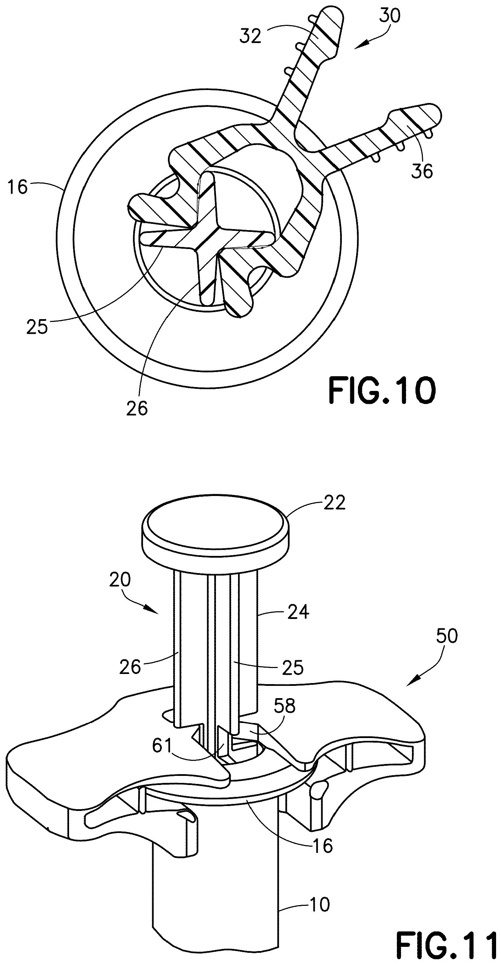

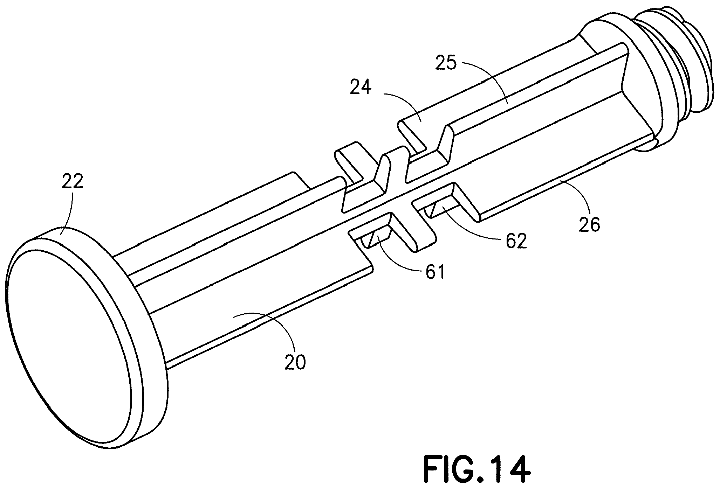

The fixation clip 50 of the third aspect of the present invention is now described in greater detail and is best seen in Figures 11 to 17. The plunger rod 20 of this design is equipped with at least one or more open slots made through the sidewalls thereof. For example, Figure 14 shows a plunger rod 20 with a first open slot 61 made in the sidewall 24. A corresponding open slot is shown as made in the sidewall 25 as well as the sidewall 26. One open slot in this design is made on all four sides of the elongated body of the plunger rod 20. The location of the open slot defines the locked position of the plunger rod 20 when assembled with the syringe barrel 10 as explained in greater detail below. To facilitate more flexible use of the kit of the present invention, additional rows of open slots 62 may be made in the sidewalls 24, 25, 26 of the plunger rod 20 along the length thereof. Each additional row of open slots would define an additional fixed position of the plunger rod inside the syringe barrel 10, which may be useful when different volumes of medical fluid need to be stored, or different headspace sizes are present, using the kit of the present invention.

The width of the open slot 61, 62 and the depth thereof are selected to be sufficiently large to accommodate a protrusion 58 of the fixation clip 50 to slide therethrough as described in greater detail below. Both the width and the depth may independently range from about 1 mm to about 10 mm, such as at least 1 mm, at least 2 mm, at least 3 mm, least 4 mm, at least 5 mm, at least 6 mm, least 7 mm, at least 8 mm, at least 9 mm, or another suitable distance. One or both dimensions of each open slot are selected to not jeopardize the mechanical integrity of the plunger rod during storage, transport, assembly, and when in use to dispense medical liquid from the syringe, as can be appreciated by those skilled in the art.

A fixation clip 50 of the third aspect of the invention comprises a top wall 52 and a bottom wall 54, which is spaced apart from and attached to the top wall 52 along a common periphery thereof. Both the top wall 52 and the bottom wall 54 define a cavity 53 formed in between thereof with a side opening in the middle portion of the fixation clip 50. To increase its mechanical integrity, additional internal sidewalls 55 may be made to connect both the top and the bottom walls together, however, these sidewalls 55 are positioned to not diminish the width of the cavity 53 below the minimally acceptable width, which is defined by the width or the diameter of the barrel flange 16.

The side opening 53 of the fixation clip 50 is made to accommodate different elements of the syringe barrel 10, barrel flange 16, and the plunger rod 20 as described below. The top wall 52 is made with a centrally located side opening having sufficient width to accept a portion of the plunger rod with the open slot 61. Two side protrusions 58 are made to narrow the side opening of the top wall 52. These side protrusions 58 are sized to pass through the open slot 61 of the plunger rod 20, as seen best in Figure 11. When the fixation clip 50 is removably placed onto the syringe barrel 10 with the plunger rod 20 inserted therein, the side protrusions 58 engage with the sidewalls 24, 25, 26 both above and below the open slot 61 and prevent the plunger rod 20 from moving along the syringe barrel 10 in either direction.

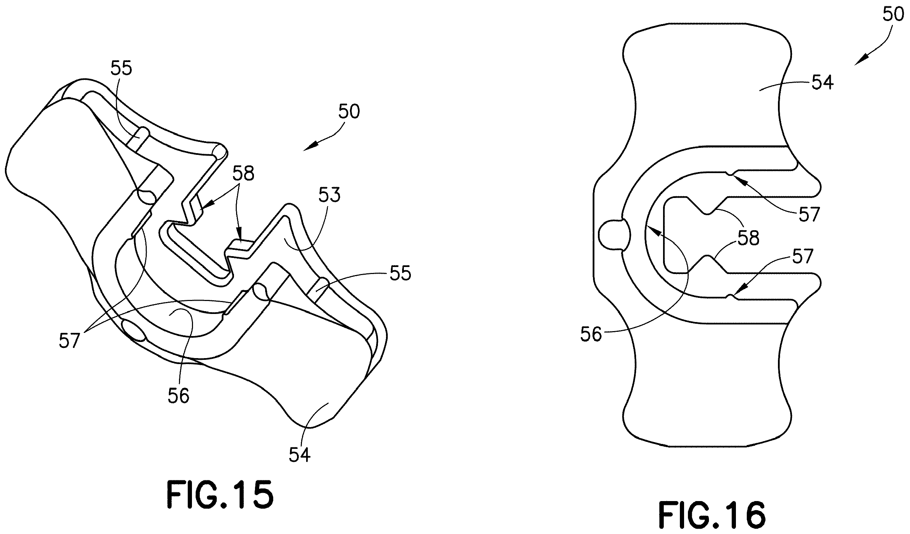

The bottom wall 54 has a larger side opening which is wide enough to accept the external surface of the cylindrical syringe barrel to rest against a semi-circular arch 56, best seen in Figures 15 and 16. The shape of the semi-circular arch 56 is made to extend from one ridge 57 on one side of the arch 56 to another ridge 57 on the other side thereof. Both ridges 57 on both sides of the arch 56 together form a snap-in detent, which is designed for the cylindrical portion of the syringe barrel 10 to removably snap into and remain in place.

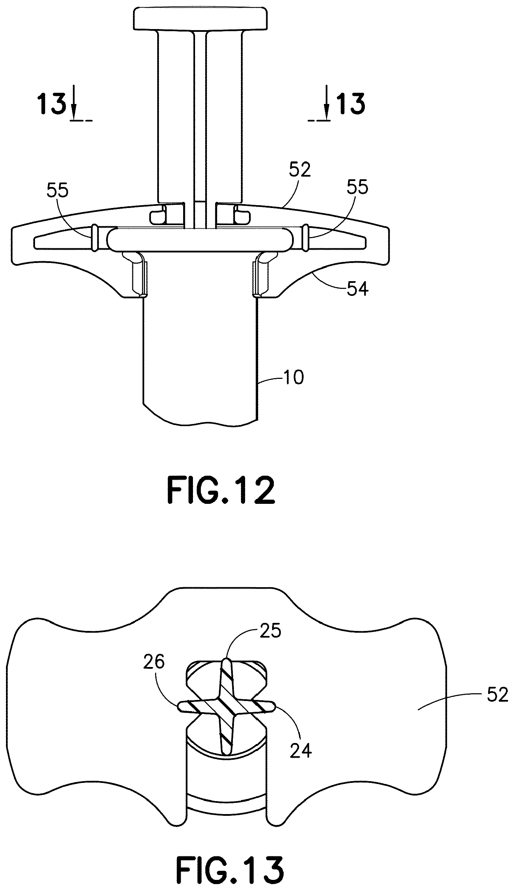

The internal cavity 53 of the fixation clip 50 is made to accept the barrel flange 16 inside thereof as seen in Figures 11 and 12. Once the fixation clip 50 is positioned over the flange 16 and snapped in place, it cannot move along the syringe barrel 10.

During the manufacturing process of the syringe assembly, the plunger rod 20 with the stopper is first placed into the syringe barrel 10. One or more open slots 61 are aligned at that point to be at or slightly above the barrel flange 16. The fixation clip 50 is then advanced from a side of the syringe assembly until the syringe barrel is snapped into the detent formed on the bottom wall 54. Positioning of the fixation clip 50 over the flange 16 provides for a lock of both the plunger rod 20 and the syringe barrel 10 to the fixation clip 50, which therefore locks all components of the kit together and prepares the syringe assembly for storage and transport.

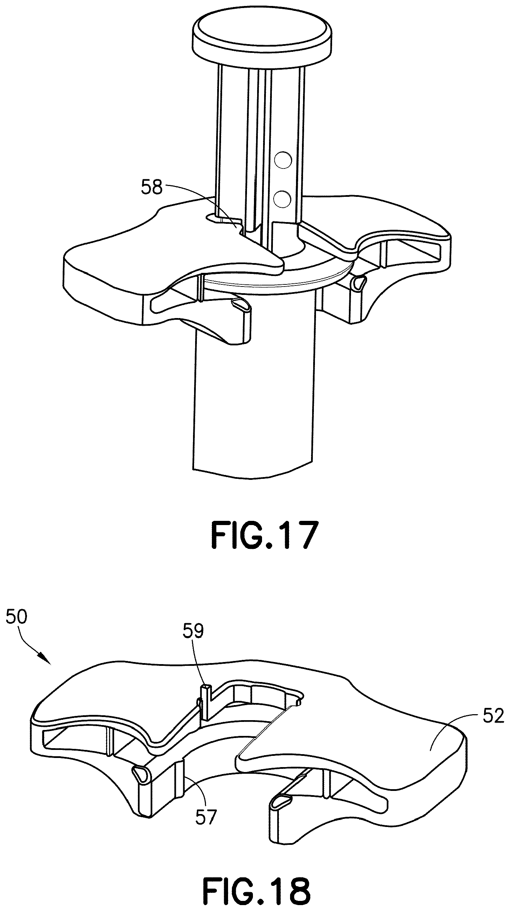

When a medical practitioner is ready to dispense the medical liquid from the syringe assembly, the practitioner rotates the plunger rod 20 so that the sidewalls 24, 25, 26 are clear of the side protrusions 58. As shown in FIG. 17, such rotation may be approximately 45° from an initial position of the plunger rod 20. The plunger rod 20 may then be pushed distally to dispense medical fluid from the syringe assembly. As the plunger rod 20 is pushed, the sidewalls 24, 25, 26 slide alongside the side protrusions 58. Accordingly, the side protrusions 58 are sized and shaped so as to fit in the spaces between adjacent sidewalls 24, 25, 26.

The fourth aspect of the present invention is now described in greater detail and illustrated in Figures 18 through 19. The fourth aspect of the invention resolves one rare circumstance that may diminish the efficacy of the third aspect of the invention, which is seen in Figure 17. If the four sidewalls of the plunger rod 20 turn in a particular way, the stopper may still find a way to move in either direction along the syringe barrel, as the sidewalls of the plunger rod 20 are no longer constrained by the fixation clip 50. That is, the plunger rod 20 may inadvertently be rotated to the position for injection, as described above with reference to Figure 17. If this happens, the V-shaped side protrusion 58 may slide along the open space of any one of the four quadrants along the plunger rod 20.

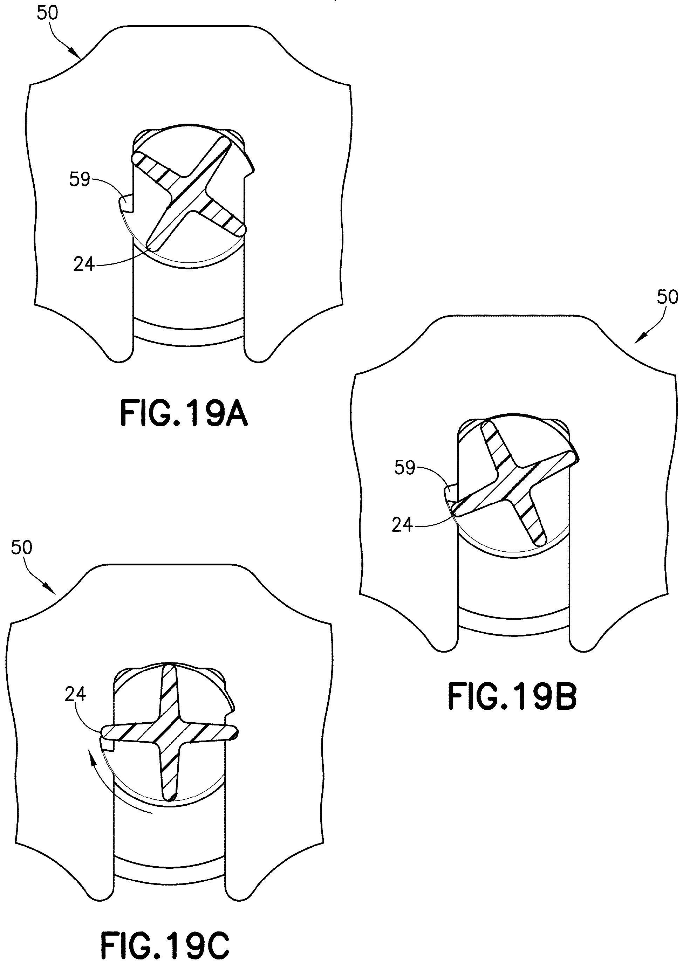

To address this issue, the fixation clip 50 of the fourth aspect of the invention features a stop 59 extending perpendicularly to and above the top wall 52, see Figure 18. The stop 59 may have a tapered ramp-in surface configured to cooperate with the respective portion of the open slot 61 in the plunger rod 20. Once the fixation clip 50 is positioned in place over the syringe assembly, the plunger rod 20 may be in one of several radial orientations as outlined in Figures 19A, 19B, and 19C. Figure 19A shows a radial orientation of the plunger rod 20 in which the sidewalls 24 of the plunger rod 20 are clear of the clip 50, allowing the plunger rod 20 to move in and out of the syringe barrel 10. Thus, the plunger rod 20 can be pushed to deliver medical fluid. To lock the syringe, the plunger rod 20 is turned clockwise causing the sidewall 24 to first engage with the stop 59 (see Figure 19B), leading to a resistance of further rotation as the stop 59 extends slightly beyond the size of the open slot 61. Continuing rotation of the plunger rod 20 leads to movement of the sidewall 24 past the stop 59, locking the plunger rod 20 in its position.

Turning the plunger rod 20 in the opposite direction, back to the orientation shown in Figure 19A, unlocks the plunger rod 20 and allows dispensing of the medical liquid from the syringe assembly by pushing the plunger rod 20.

While this disclosure has been described as having exemplary designs, the present disclosure can be further modified within the spirit and scope of this disclosure. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims.

## Claims

1. A fixation clip for use with a plunger assembly and a corresponding syringe barrel, said plunger assembly including a plunger stopper connected to a plunger rod, , wherein the fixation clip comprises a pair of hingedly-connected arms forming a normally-closed damp with at least one or more notches formed on one or both of the arms of the fixation clip, thereby the fixation clip is configured for removable attachment to and engagement with the plunger rod,
wherein placement of the fixation clip onto the plunger rod adjacent a flange of the syringe barrel causes the fixation clip to extend outwardly from the plunger rod beyond the syringe barrel, which in turn prevents the plunger rod and the stopper from unintended longitudinal movement along the syringe barrel.

2. A prefillable syringe comprising:
a syringe barrel comprising a proximal end, and a flange disposed at said proximal end,
a plunger rod for attachment with a stopper for use with the syringe barrel, the plunger rod comprising an elongated body having a distal attachment end configured for attachment to the stopper and a proximal end, and the elongated body extending along a longitudinal axis between the distal end and the proximal end, the plunger rod further comprising one or more ridges extending sideways from the elongated body and spaced apart along thereof, and
a fixation clip comprising one or more notches matching in size and spacing to the one or more ridges of the plunger rod, the fixation clip configured to removably attach to the plunger rod such that one or more ridges of the plunger rod are positioned within one or more notches of the fixation clip,
wherein placement of the fixation clip onto the plunger rod adjacent to the syringe barrel flange causes the fixation clip to extend outwardly from the plunger rod beyond the syringe barrel, which in turn prevents the plunger rod and the stopper from unintended longitudinal movement along the syringe barrel.

3. A kit for preserving sterility of a prefillable syringe, the kit comprising:
a syringe barrel,
a plunger rod for attachment with a stopper for use with the syringe barrel, the plunger rod comprises an elongated body having a distal attachment end configured for attachment to the stopper and a proximal end, and the elongated body extending along a longitudinal axis between the distal end and the proximal end, the plunger rod further comprising one or more ridges extending sideways from the elongated body and spaced apart along thereof, and
a fixation clip comprising one or more notches matching in size and spacing to the one or more ridges of the plunger rod, wherein the fixation clip is configured to removably attach to the plunger rod such that one or more ridges of the plunger rod are positioned within one or more notches of the fixation clip,
whereby upon inserting the plunger rod and the stopper into the syringe barrel and filling the syringe with a medical liquid, placement of the fixation clip onto the plunger rod adjacent the syringe barrel causes the fixation clip to extend outwardly from the plunger rod beyond the syringe barrel, which in turn prevents the plunger rod and the stopper from unintended longitudinal movement along the syringe barrel.

4. The kit as in claim 3, wherein the elongated body of the plunger rod comprises four sidewalls extending radially away from a center and forming an X-shaped cross-sectional profile with the sidewalls positioned at 90 degrees to each other and defining four quadrants in between, the one or more ridges extending sideways in at least one quadrant.

5. The kit as in claim 4, wherein a plurality of the ridges are located along the plunger rod in at least one of: the same quadrant of the elongated body; at least two opposing quadrants of the elongated body of the plunger rod; and all four quadrants of the elongated body of the plunger rod.

6. The kit as in claim 4, wherein each ridge has a triangular shape spanning from one sidewall of the elongated body to the adjacent sidewall in the same quadrant thereof, and wherein the triangular shape of each ridge is defined by a first side and a second side between adjacent sidewalls of the plunger rod, wherein the first side faces the proximal end of the plunger rod and the second side faces the distal end of the plunger rod.

7. The kit as in claim 3, wherein the fixation clip comprises a pair of hingedly-connected V-shaped arms, each arm defining a first end and a second end, wherein the one or more notches are positioned at the first end of at least one of the arms.

8. The kit as in claim 3, wherein the fixation clip is made as a single body with the arms connected by a living hinge so that moving of the second end of each arm toward each other causing separation of respective first ends of each arm away from each other.

9. The kit as in claim 3, wherein each notch is made with dimensions matching or exceeding dimensions of the corresponding ridge on the plunger rod.

10. The kit as in claim 9, wherein the notch is made wider than the corresponding ridge, thereby forming a gap in between upon placement of the fixation clip over the plunger rod.

11. The kit as in claim 3, wherein the syringe barrel is equipped with a barrel flange at a proximal open end thereof, the fixation clip further comprises a flange-engaging extension portion extending from one or both second ends of the pair of arms forming the fixation clip.

12. The kit as in claim 11, wherein the fixation clip further defining a cutout slot formed next to the extension portion thereof and sized to accept the flange of the syringe barrel therein upon placement of the fixation clip over the plunger rod of the kit.

13. The kit as in claim 12, wherein the flange-engaging extension portion of the fixation clip further comprises a curved rest pad sized to be positioned next to an external surface of the syringe barrel.

14. A kit for preserving sterility of a prefillable syringe, the kit comprising:
a syringe barrel comprising a proximal end, and a flange disposed at said proximal end,
a plunger rod for attachment with a stopper for use with the syringe barrel, the plunger rod comprising an elongated body having a distal attachment end configured for attachment to the stopper and a proximal end, and the elongated body having a plurality of sidewalls extending along a longitudinal axis between the distal end and the proximal end, the plunger rod further comprising one or more notches formed in each of the plurality of sidewalls of the elongated body and spaced apart along thereof, and
a fixation clip comprising protrusions receivable within the one or more ridges of the plunger rod, the fixation clip configured to removably attach to the plunger rod such that the protrusions are positioned within respective notches of the one or more notches formed in each of the plurality of sidewalls,
wherein placement of the fixation clip onto the plunger rod adjacent to the syringe barrel flange causes the fixation clip to extend outwardly from the plunger rod beyond the syringe barrel, which in turn prevents the plunger rod and the stopper from unintended longitudinal movement along the syringe barrel.

15. The kit as in claim 14, wherein the fixation clip comprises a top wall and a bottom wall, with the protrusions formed on the top wall, and wherein the top wall is spaced apart longitudinally from the bottom wall to form a cavity configured to receive the flange when the fixation clip is placed onto the plunger rod, to prevent longitudinal movement of the fixation clip relative to the syringe barrel.
